(19) Europäisches Patentamt
European Patent Office
Office européen des brevets

(11) EP 1 698 654 A1

(12) **EUROPEAN PATENT APPLICATION**
published in accordance with Art. 158(3) EPC

(43) Date of publication:
**06.09.2006 Bulletin 2006/36**

(21) Application number: **04807653.3**

(22) Date of filing: **24.12.2004**

(51) Int Cl.:
***C08J 5/12*** (2006.01) ***C08J 7/00*** (2006.01)
***C08J 7/02*** (2006.01) ***A61M 5/14*** (2006.01)
*C08L 9/00* (2006.01)

(86) International application number:
**PCT/JP2004/019296**

(87) International publication number:
**WO 2005/063859 (14.07.2005 Gazette 2005/28)**

(84) Designated Contracting States:
**DE**

(30) Priority: **26.12.2003 JP 2003433773**

(71) Applicant: **JSR Corporation**
**Tokyo 104-8410 (JP)**

(72) Inventors:
- **FURUICHI, Minoru,**
  **c/o JSR CORPORATION**
  **Tokyo 104-8410 (JP)**
- **TOYOTA, Nobuyuki,**
  **c/o JSR CORPORATION**
  **Tokyo 104-8410 (JP)**
- **AOYAMA, Teruo,**
  **c/o JSR CORPORATION**
  **Tokyo 104-8410 (JP)**

(74) Representative: **TBK-Patent**
**Bavariaring 4-6**
**80336 München (DE)**

# (54) METHOD FOR ADHERING POLYBUTADIENE FORMED ARTICLE, POLYBUTADIENE COMPOSITE FORMED ARTICLE MANUFACTURED THEREBY, MEDICAL MEMBER, AND INFUSION FLUID SET

(57) To improve the joining (adhesion) force of a polybutadiene formed article and a polar resin formed article, and to further improve the joining force by selecting (combining) a solvent(s) for these formed articles.

A method for adhering a polybutadiene formed article, which comprises (1) the step of reducing the water contact angle of a surface of the polybutadiene formed article by ozone treatment, electron beam treatment, corona discharge treatment, plasma discharge treatment, ultraviolet laser treatment or chemical treatment, and (2) the step of adhering the polybutadiene formed article reduced in the water contact angle to a polar resin formed article.

Figure 1

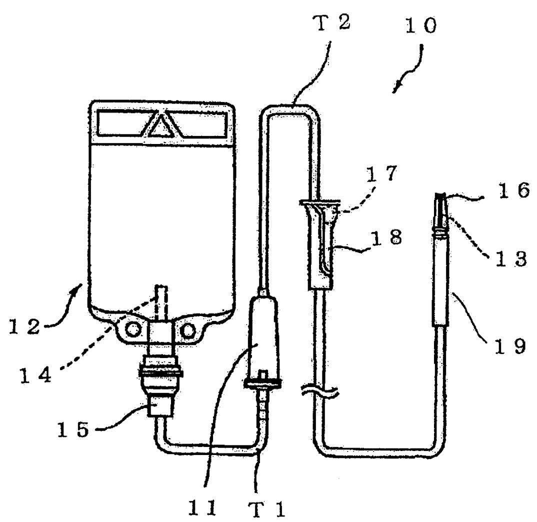




Printed by Jouve, 75001 PARIS (FR)

## Description

Technical Field

The present invention relates to a method for adhering a polybutadiene formed article, a polybutadiene composite formed article obtained therefrom, a medical member and an infusion set.

Background Art

In recent years, polybutadiene represented by syndiotactic 1,2-polybutadiene (RB) has attracted attention as an alternative material for PVC (vinyl chloride-based resin), in which no plasticizer is used, and the present inventors have proposed a medical member in which an RB tube and an RB connector are adhered to each other (patent documents 1: JP-A-2004-321788), and the like.
By the way, an infusion set is commercialized by solvent joining (adhesion) of a tube and a connector. Until now, the above-mentioned infusion set has been commercialized by a PVC tube/a solvent (polar solvent)/a polar resin connector. However, the movement of avoiding PVC becomes remarkable, and in recent years, studies of RB have increased in place of the PVC tube, as described above. However, RB is poor in polarity, so that the joining according to the polar solvent/the polar resin is insufficient depending on its use in some cases.
In particular, in Japan or the U.S., when an intravenous drip injection is given to a patient using the infusion set, a pump is occasionally used. In this case, a pressure is applied to the infusion set, so that, for example, there is the possibility of generating a leak at a joint of the tube and the connector.
[Patent document 1] JP-A-2004-321788

Disclosure of the Invention

Problem That the Invention Is to Solve

An object of the invention is to improve the joining (adhesion) force of a polybutadiene formed article and a polar resin formed article, and to further improve the joining force by selecting (or combining) a solvent(s) for these formed articles.

Means for Solving the Problem

The invention relates to a method for adhering a polybutadiene formed article, which comprises

(1) the step of reducing the water contact angle of a surface of the polybutadiene formed article (hereinafter also referred to as "step (1)"), and
(2) the step of adhering the above-mentioned polybutadiene formed article which is reduced in the water contact angle to a polar resin formed article (hereinafter also referred to as "step (2)").

The above-mentioned polybutadiene as used herein is preferably syndiotactic 1,2-polybutadiene having a crystallinity of 5% or more.
Further, the above-mentioned step (1) includes at least one selected from the group of ozone treatment, electron beam treatment, corona discharge treatment, plasma discharge treatment, radiation (X-ray, $\alpha$-ray, $\beta$-ray) treatment, ultraviolet treatment, ultraviolet laser treatment and chemical treatment.
The water contact angle ($CA_{BR}$) of the water contact angle-reduced polybutadiene formed article which is obtained in the above-mentioned step (1) is 80 degrees or less.
The above-mentioned polar resins include at least one selected from the group of a polycarbonate resin, a polyester resin, an ABS resin, a polystyrene resin, a polyurethane resin, a polyamide resin, a polyalkyl acrylate resin, a polyalkyl methacrylate resin, a polyvinyl acetate resin, polyvinyl chloride and a polyvinylidene chloride resin.
The difference ($\Delta CA$) between the water contact angle ($CA_{BR}$) of the water contact angle-reduced polybutadiene formed article obtained in the above-mentioned step (1) and the water contact angle ($CA_{PR}$) of the polar resin formed article is from +60 degrees to -15 degrees.
The adhesion in the above-mentioned step (2) is preferably adhesion by the use of an organic solvent.
The above-mentioned organic solvents preferably include at least one selected from the group of cyclohexanone, tetrahydrofuran, cyclohexane, methyl ethyl ketone, acetone and ethyl acetate.
On the occasion of the adhesion in step (2), it is preferred that the water contact angle-reduced polybutadiene formed article which is obtained in the above-mentioned step (1) and the polar resin formed article are previously treated with

the above-mentioned organic solvent.
Then, the invention relates to a polybutadiene composite formed article obtained by the above-mentioned method for adhering a polybutadiene formed article.
Further, the invention relates to a medical member which contains at least the above-mentioned polybutadiene composite formed article.
Furthermore, the invention relates to an infusion set having the above-mentioned medical member as a constituent element.

Advantages of the Invention

According to the invention, polar groups are planted on a surface of the above-mentioned polybutadiene formed article, or the surface is roughened to reduce the water contact angle of the surface of the polybutadiene formed article, thereby being able to improve the joining (adhesion) force with the polar resin formed article. Moreover, the solvent (s) for these formed articles is selected (combined), thereby being able to further improve the joining force.

Brief Description of the Drawings

[Fig. 1] Fig. 1 is a plan view showing an infusion set having medical members of the present invention as constituent elements.
[Fig. 2] Fig. 2 is a schematic view showing (a) a connector and (b) a tube.

Description of Reference Numerals and Signs

10 Infusion Set
11 Drip Chamber
12 Infusion Bag
13 Puncture Needle
14 Tube for Discharging Infusion Solution
15 Connecting Member (Connector)
16 Cap
17 Roller
18 Roller Clamp
19 Joining Member
T1, T2 Tubes

Best Mode for Carrying Out the Invention

<u>Polybutadiene Formed Article</u>

As polybutadiene which constitutes the polybutadiene formed article of the invention such as a tube, there is preferably used syndiotactic 1,2-polybutadiene (A) alone or a composition of syndiotactic 1,2-polybutadiene (A) and other thermoplastic polymer (B). This syndiotactic 1,2-polybutadiene (A) is syndiotactic 1,2-polybutadiene having a crystallinity of 5% or more, preferably 10 to 40%, and the melting point thereof ranges preferably from 50 to 150°C, and more preferably from 60 to 140°C. The crystallinity and melting point within these ranges result in an excellent balance between mechanical strength such as tensile strength or tearing strength and flexibility.
Syndiotactic 1, 2-polybutadiene having a crystallinity of up to about 5 to 25% by weight (hereinafter also referred to as "low crystalline RB") is excellent in flexibility, so that it is used as a main part of the tube. However, the low crystalline RB is inferior in steam sterilization resistance because of its low melting point (melting point = about 70 to 95°C). It is therefore desirable to crosslink it by electron beam irradiation, thereby giving heat resistance, as described later.
On the other hand, syndiotactic 1,2-polybutadiene having a crystallinity of about 25 to 40% by weight (hereinafter also referred to as "high crystalline RB ") is relatively high in melting point (melting point = about 105 to 140°C), but high in hardness and inferior in flexibility. Accordingly, this can be preferably used as the connector.
For example, syndiotactic 1,2-polybutadiene having a 1,2-bond content of 70% or more is used as syndiotactic 1,2-polybutadiene (A) in the present invention, and obtained, for example, by polymerizing butadiene under the presence

of a catalyst containing a cobalt compound and an aluminoxane. However, the production method should not be construed as being limited thereto.

The 1,2-bond content in butadiene bond units of syndiotactic 1,2-polybutadiene (A) used in the invention is usually 70% or more, preferably 80% or more, and more preferably 90% or more. When the 1, 2-bond content is 70% or more, the 1,2-polybutadiene exhibits properties as a good thermoplastic elastomer.

Syndiotactic 1,2-polybutadiene (A) used in the invention may be copolymerized with a small amount of a conjugated diene other than butadiene. The conjugated dienes other than butadiene include 1,3-pentadiene, a higher alkyl group-substituted 1,3-butadiene derivative, a 2-alkyl-substituted 1,3-butadiene and the like.
Of these, the higher alkyl group-substituted 1,3-butadiene derivatives include 1-pentyl-1,3-butadiene, 1-hexyl-1,3-butadiene, 1-heptyl-1,3-butadiene, 1-octyl-1,3-butadiene and the like.

Here, the typical examples of the 2-alkyl-substituted 1,3-butadienes include 2-methyl-1,3-butadiene (isoprene), 2-ethyl-1,3-butadiene, 2-propyl-1,3-butadiene, 2-isopropyl-1,3-butadiene, 2-butyl-1,3-butadiene, 2-isobutyl-1,3-butadiene, 2-amyl-1,3-butadiene, 2-isoamyl-1,3-butadiene, 2-hexyl-1,3-butadiene, 2-cyclohexyl-1,3-butadiene, 2-isohexyl-1,3-butadiene, 2-heptyl-1,3-butadiene, 2-isoheptyl-1,3-butadiene, 2-octyl-1,3-butadiene, 2-isooctyl-1,3-butadiene and the like. Of these conjugated dienes, the preferred conjugated dienes copolymerized with butadiene include isoprene and 1,3-pentadiene. The content of butadiene in the monomer components subjected to polymerization is preferably 50 mol% or more, and particularly preferably 70 mol% or more.

Syndiotactic 1,2-polybutadiene (A) used in the invention is obtained by polymerizing butadiene, for example, under the presence of the catalyst containing the cobalt compound and the aluminoxane, as described above. The above-mentioned cobalt compounds include preferably an organic acid salt of cobalt having 4 or more carbon atoms. Specific examples of the organic acid salts of cobalt include a butyrate, a hexanoate, a heptylate, an octylate such as 2-ethylhexyli acid, a decanoate, a salt of a higher fatty acid such as stearic acid, oleic acid or erucic acid, a benzoate; an alkyl-, aralkyl- or allyl-substituted benzoate such as a tolylate, a xylylate or an ethylbenzoic acid, a naphthoate and an alkyl-, aralkyl- or allyl-substituted naphthoate. Of these, 2-ethylhexylic acid or a so-called octylate, a stearate and a benzoate are preferred for excellent solubility in a hydrocarbon solvent.

The above-mentioned aluminoxanes include, for example, one represented by the following general formula (I) or general formula (II):

[Formula 1]

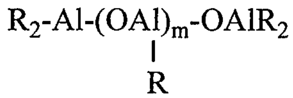

-------(I)

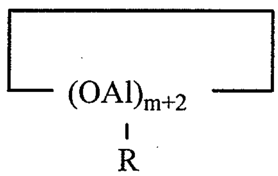

-----(II)

In the aluminoxane represented by general formula (I) or (II), R is a hydrocarbon group such as a methyl group, an ethyl group, a propyl group or a butyl group, preferably a methyl group or an ethyl group, and particularly preferably a methyl group. Further, m is an integer of 2 or more, preferably an integer of 5 or more, and more preferably an integer of 10 to 100. Specific examples of the aluminoxanes include methylaluminoxane, ethylaluminoxane, propylaluminoxane, butylaluminoxane and the like, and methylaluminoxane is particularly preferred.

It is very preferred that the polymerization catalyst contains a phosphine compound, in addition to the above-mentioned cobalt compound and aluminoxane. The phosphine compound is a component effective for activation of the polymerization catalyst, and the control of the vinyl bond structure and crystallinity. It includes preferably an organic phosphorus compound represented by the following general formula (III):

$$P(Ar)_n(R')_{3-n} \qquad (III)$$

In general formula (III), Ar represents a group shown below:

[Formula 2]

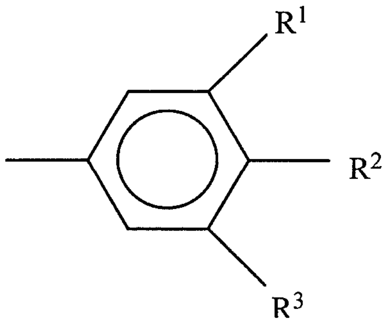

(In the above-mentioned group, $R^1$, $R^2$ and $R^3$, which may be the same or different, each represent a hydrogen atom, an alkyl group preferably having 1 to 6 carbon atoms, a halogen atom, an alkoxyl group preferably having 1 to 6 carbon atoms or an aryl group preferably having 6 to 12 carbon atoms.)

Further, in general formula (III), R' indicates a cycloalkyl group or an alkyl-substituted cycloalkyl group, and n is an integer of 0 to 3.

Specific examples of the phosphine compounds represented by general formula (III) include tri-(3-methylphenyl)-phosphine, tri(3-ethylphenyl)phosphine, tri(3,5-dimethylphenyl)phosphine, tri(3,4-dimethylphenyl)phosphine, tri(3-isopropylphenyl)phosphine, tri(3-t-butylphenyl)phosphine, tri(3,5-diethylphenyl)phosphine, tri(3-methyl-5-ethylphenyl) phosphine, tri(3-phenylphenyl)phosphine, tri(3,4,5-trimethylphenyl)phosphine, tri(4-methoxy-3,5-dimethylphenyl)phosphine, tri(4-ethoxy-3,5-diethylphenyl)phosphine, tri(4-butoxy-3,5-dibutylphenyl)phosphine, tri(p-methoxyphenyl)phosphine, tricyclohexylphosphine, dicyclohexylphenylphosphine, tribenzylphosphine, tri(4-methylphenylphosphine), tri(4-ethylphenylphosphine) and the like. Of these, particularly preferred examples thereof include triphenylphosphine, tri(3-methylphenyl)phosphine, tri(4-methoxy-3,5-dimethylphenyl)phosphine and the like.

Further, as the cobalt compound, there can be used a compound represented by the following general formula (IV):

[Formula 3]

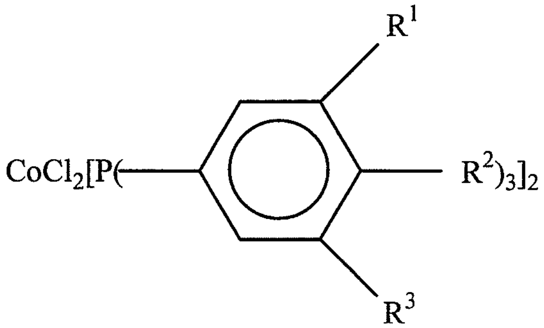

------(IV)

The compound represented by the above-mentioned general formula (IV) is a complex having a phosphine compound in which n is 3 in the above-mentioned general formula (III), as a ligand to cobalt chloride. When this cobalt compound is used, one previously synthesized may be used, or a method of contacting cobalt chloride with the phosphine compound in a polymerization system may be used. The amount of 1,2-vinyl bonds and crystallinity of the resulting syndiotactic 1,2-polybutadiene can be controlled by variously selecting the phosphine compound in the complex.

Specific examples of the cobalt compounds represented by the above-mentioned general formula (IV) include

cobalt bis(triphenylphosphine)dichloride, cobalt bis[tris (3-methylphenylphosphine)]dichloride, cobalt bis[tris(3-ethylphenylphosphine)]dichloride, cobalt bis[tris(4-methylphenylphosphine)]dichloride, cobalt bis[tris(3,5-dimethylphenylphosphine)]dichloride, cobalt bis[tris(3,4-dimethylphenylphosphine)]dichloride, cobalt bis[tris(3-isopropylphenylphosphine)] dichloride, cobalt bis[tris(3-t-butylphenylphosphine)]dichloride, cobalt bis[tris(3,5-diethylphenylphosphine)]dichloride, cobalt bis[tris(3-methyl-5-ethylphenylphosphine)]dichloride, cobalt bis[tris(3-phenylphenylphosphine)]dichloride, cobalt bis[tris(3,4,5-trimethylphenylphosphine)]dichloride, cobalt bis[tris(4-methoxy-3,5-dimethylphenylphosphine)]dichloride, cobalt bis[tris(4-ethoxy-3,5-diethylphenylphosphine)]dichloride, cobalt bis[tris(4-butoxy-3,5-dibutylphenylphosphine) dichloride, cobalt bis[tris(4-methoxyphenylphosphine)]dichloride, cobalt bis[tris(3-methoxyphenylphosphine)]dichloride, cobalt bis[tris(4-dodecylphenylphosphine)]dichloride, cobalt bis[tris(4-ethylphenylphosphine)]dichloride and the like.

Of these, particularly preferred are cobalt bis(triphenylphosphine)dichloride, cobalt bis[tris(3-methylphenylphosphine)]dichloride, cobalt bis[tris(3,5-dimethylphenylphosphine)]dichloride, cobalt bis[tris(4-methoxy-3,5-dimethylphenylphosphine)]dichloride and the like.

As the amount of the catalyst used, the cobalt compound is used in an amount of 0.001 to 1 mmol, preferably about 0.01 to about 0.5 mmol, in terms of a cobalt atom per mole of butadiene for homopolymerization of butadiene, and per mole of the total amount of butadiene and a conjugated diene other than butadiene for copolymerization. Further, the amount of the phosphine compound used is usually from 0.1 to 50, preferably from 0.5 to 20, and more preferably from 1 to 20, as the atomic ratio of phosphorus to cobalt (P/Co). Furthermore, the amount of the aluminoxane used is usually from 4 to $10^7$, and preferably from 10 to $10^6$, as the atomic ratio of aluminum to cobalt of the cobalt compound (Al/Co). When the complex represented by general formula (IV) is used, the amount of the phosphine compound used is 2 as the atomic ratio of phosphorus to cobalt (P/Co), and the amount of the aluminoxane used follows the above description.

Inert organic solvents used as polymerization solvents include, for example, aromatic hydrocarbon solvents such as benzene, toluene, xylene and cumene, aliphatic hydrocarbon solvents such as n-pentane, n-hexane and n-butane, alicyclic hydrocarbon solvents such as cyclopentane, methylcyclopentane and cyclohexane, and mixtures thereof.

The polymerization temperature is usually from -50 to 120°C, and preferably from -20 to 100°C.

The polymerization reaction may be either batch-wise or continuous. The monomer concentration in the solvent is usually from 5 to 50% by weight, and preferably from 10 to 35% by weight.

Further, for producing the polymer, it is necessary to take into consideration that contamination with a compound having an inactivating function, such as oxygen, water or carbon dioxide, in a polymerization system should be decreased to the utmost, in order not to inactivate the catalyst and polymer of the invention. When the polymerization reaction proceeds to a desired stage, an alcohol, another polymerization terminator, an antiaging agent, an antioxidant, an ultraviolet absorber and the like are added to the reaction mixture, and then, the polymer formed is separated, washed and dried according to conventional methods. Thus, the syndiotactic 1,2-polybutadiene used in the invention can be obtained.

The weight average molecular weight of syndiotactic 1,2-polybutadiene (A) used in the invention is preferably from 10, 000 to 5, 000, 000, more preferably from 10, 000 to 1, 500, 000, and particularly preferably from 50, 000 to 1, 000, 000. When the weight average molecular weight is less than 10,000, fluidity is extremely high, unfavorably resulting in very difficult processing and the formation of a sticky formed article (medical member). On the other hand, exceeding 5,000,000 results in extremely low fluidity, which unfavorably makes processing very difficult.

On the other hand, thermoplastic polymer (B) is a thermoplastic resin and/or thermoplastic elastomer other than the above-mentioned component (A), and specifically, at least one selected from the group consisting of polyethylene, polypropylene, a styrene-butadiene-styrene block copolymer (SBS), a styrene-isoprene-styrene block copolymer (SIS), hydrogenated polymers thereof (SEBS and SEPS), polybutadiene (BR) other than the above-mentioned syndiotactic 1,2-polybutadiene, an ABS resin, polyisoprene, various polyethylenes (LLDPE, ULDPE and LDPE), an ethylene-vinyl acetate copolymer, an ethylene-acrylate copolymer and an ethylene-methacrylate copolymer.

The amount of component (B) blended is 40 parts by weight or less, and preferably from 0 to 35 parts by weight, based on 100 parts by weight of the total amount of components (A) and (B). Exceeding 40 parts by weight results in a decrease in the ratio of component (A) used. As a result, the original flexibility of component (A) is lost.

Further, the composition used in the present invention may contain an additive such as a lubricant, a filler or a foaming agent, in addition to the above-mentioned components (A) and (B), as needed. Specific examples of the above-mentioned additives include fillers such as talc, silica, magnesium hydroxide, calcium carbonate, glass, carbon fiber and glass balloons, and foaming agents such as Microsphere manufactured by Matsumoto Yushi-Seiyaku Co., Ltd., ADCA, OBSH, sodium bicarbonate and AIBN, as well as lubricants such as paraffin oil, silicone oil, liquid polyisoprene, liquid butadiene, erucic acid amide and stearic acid amide.

The amount of the lubricant used is 10 parts by weight or less, and preferably from 0.01 to 8 parts by weight, based on 100 parts by weight of the resin components, namely the total of components (A) and (B). When it exceeds 10 parts by weight, the lubricant unfavorably bleeds out from a product to seep into a drug used.

Further, in order to improve the balance between heat resistance and flexibility by electron beam irradiation,

another additive, for example, a multifunctional monomer such as trimethylolpropane trimethacrylate, a photopolymerization initiator such as hydroxycyclohexyl phenyl ketone, or a photosensitizer such as benzophenone may be added in an amount of 5 parts by weight or less based on 100 parts by weight of the syndiotactic 1,2-polybutadiene.

Preparation and Forming of Composition

The composition used for the polybutadiene formed article in the invention comprising the above-mentioned component (A) alone or components (A) and (B) to which the above-mentioned additive(s) and the like are added as needed is softened by heating, kneaded and formed. Kneading and forming are conducted at a temperature equal to or higher than the softening temperature or melting temperature of the syndiotactic 1,2-polybutadiene, at which good formability is exhibited, thereby providing a homogeneous formed article (medical member such as the tube). Accordingly, the forming temperature is preferably from about 90 to about 170°C. In order to obtain the formed article such as the tube or the connector, press molding, extrusion molding, injection molding, blow molding, profile extrusion molding, T-die film molding, inflation molding, powder slush molding, rotational molding or the like is utilized, thereby molding the composition to the tube or the like or the connector having a tube connecting portion.

Electron Beam Irradiation

Of polybutadiene formed articles of the invention, the tube requires flexibility, so that low crystalline RB is used. However, the melting point thereof is low. Accordingly, in order to allow it to exhibit steam sterilization resistance, it can be crosslinked by subsequent electron beam irradiation. On the electron beam irradiation, a three-dimensional crosslinked structure is formed by radical polymerization of a vinyl group of the syndiotactic 1, 2-polybutadiene to give heat resistance to the formed article (tube). An electron beam has permeability to a synthetic resin, and the degree of its permeation depends on the thickness of the formed article and kinetic energy of the electron beam.
When energy of the electron beam is controlled permeably uniformly in the thickness direction according to irradiation thickness, the formed article (tube) whose degree of crosslinking is homogenized in the thickness direction can be obtained.
The connector may also be subjected to the electron beam irradiation.
Further, the electron beam irradiation may be carried out either before or after adhesion of the tube to the connector.
The energy of the electron beam is preferably from 50 to 3,000 kV, and more preferably from 300 to 2,000 kV, to the above-mentioned polybutadiene formed article (medical member) such as the tube. The energy less than 50 kV results in a relative increase in the ratio of electrons captured and absorbed by a surface layer portion to decrease the electron beam which permeates through the formed article. Accordingly, the inside thereof is crosslinked late compared to the surface layer portion, which unfavorably causes the difference in the degree of crosslinking. On the other hand, exceeding 3, 000 kV results in too high a degree of crosslinking, thereby making the formed article so hard as to unfavorably cause low elasticity and elongation.
Further, the dose of the electron beam in this case is preferably from 1 to 100 Mrad (corresponding to 10 to 1,000 kGy in the SI unit system), and more preferably from 1 to 50 Mrad. Less than 1 Mrad results in a decrease in the degree of crosslinking of 1,2-polybutadiene, whereas exceeding 100 Mrad results in too high a degree of crosslinking, thereby making the formed article hard, which unfavorably causes low elasticity and elongation.
The effect of crosslinking by electron beam irradiation can be expressed by the product of electron beam energy and dose. In the invention, the product of electron beam acceleration voltage (kV) and irradiation dose (Mrad) is preferably from 2,000 to 20,000 (kV·Mrad), and more preferably from 5,000 to 16, 000 (kV·Mrad). Less than 2, 000 (kV·Mrad) results in a relative increase in the ratio of electrons captured and absorbed by the surface layer portion to decrease the electron beam which permeates through the polybutadiene formed article (medical member). Accordingly, the inside thereof is crosslinked late compared to the surface layer portion, which unfavorably causes the difference in the degree of crosslinking. On the other hand, exceeding 20,000 (kV·Mrad) results in too high a degree of crosslinking, thereby making the formed article so hard as to unfavorably cause low elasticity and elongation.
By applying the electron beam irradiation as described above to the polybutadiene formed article (medical member such as the tube) of the invention, the degree of elasticity at an elongation of 50% of the medical member after the electron beam irradiation ($M2_{50}$) can be increased to preferably 1.1 to 2.5 times, more preferably 1.1 to 2.0 times the degree of elasticity at an elongation of 50% before the electron beam irradiation ($M1_{50}$). When $M2_{50}/M1_{50}$ is less than 1.1, the electron beam crosslinking does not proceed, resulting in poor steam sterilization resistance. On the other hand, when it exceeds 2.5, the polybutadiene formed article (medical member such as the tube) crosslinked becomes too hard, unfavorably resulting in the loss of flexibility. $M2_{50}/M1_{50}$ can be easily controlled by adjusting the above-mentioned product of electron beam acceleration voltage (kV) and irradiation dose (Mrad) to 2, 000 to 20,000 (kV·Mrad).
Further, the thus-obtained polybutadiene formed article (medical member) crosslinked after the electron beam irradiation has steam sterilization resistance. For example, even when using the crosslinked infusion tube of the invention,

sterilization by steam is performed at a temperature of 100 to 121°C for about 10 to about 20 minutes, deformation does not happen to occur.

The term "steam sterilization resistance" as used herein means that when the formed article of a resin such as the infusion tube (for example, a tube having an internal diameter of 3 mm, an outer diameter of 4.4 mm, a wall thickness of 0.7 mm and a tube length of 20 cm) is placed in a high-pressure steam sterilizer and sterilized by steam at 121°C for 20 minutes, the circular shape before sterilization is kept and no deformation is observed.

Further, the haze value of the polybutadiene formed article (medical member such as the tube) of the invention irradiated with the electron beam is preferably 30 or less, and more preferably 25 or less. The haze value is the measure of transparency, and the transparency is improved with a decrease in this value. This haze value was measured in accordance with ASTM D-1003.

Furthermore, the polybutadiene formed article (medical member such as the tube) of the invention after the electron beam irradiation has a toluene insoluble of usually 50 to 99% by weight, preferably 80 to 95% by weight. The toluene insoluble is the barometer showing to what degree double bonds in syndiotactic 1,2-polypropylene (A) have been crosslinked.

As for the toluene insoluble as used herein, the polybutadiene formed article (medical member) of the invention ((a) g) was immersed in 100 ml of toluene, allowed to stand at 30°C for 48 hours, and then, filtered using a 100-mesh filter. After a part ((c) ml) of the filtrate was collected, it was evaporated to dryness, the resulting residual solid matter (toluene soluble: (b) g) was weighed, and the gel content was calculated by the following equation:

$$\text{Gel content (\% by weight)} = [\{a - b \times (100/c)\}/a] \times 100$$

When the toluene insoluble is less than 50% by weight, crosslinking by the electron beam irradiation is insufficient, which causes poor heat resistance to result in poor steam sterilization resistance. On the other hand, when it exceeds 99% by weight, crosslinking by the electron beam irradiation proceeds too much. As a result, the medical member becomes too hard, unfavorably resulting in the loss of flexibility.

The above-mentioned toluene insoluble can be easily controlled by adjusting the above-mentioned product of electron beam acceleration voltage (kV) and irradiation dose (Mrad) to 2,000 to 20,000 (kV·Mrad).

Further, the polybutadiene formed article (medical member such as the tube) of the invention has a halogen atom content of preferably 200 ppm or less, more preferably 100 ppm or less. As for this halogen atom content, for example, by using the inert organic solvent of the non-halogen family as the polymerization solvent as described above, the halogen atom content in 1, 2-polybutadine obtained can be adjusted preferably to 200 ppm or less, and more preferably to 100 ppm or less. Further, it is preferred that only non-halogen compounds are used in the catalyst system, because the halogen atom content of the polybutadiene formed article (medical member) can be further reduced.

The polybutadiene formed article thus subjected to the electron beam irradiation is excellent in flexibility and hardness, and has steam sterilization resistance, so that it is also useful for the connector as well as for the tube.

The polybutadiene formed article used in the invention indicates a tube formed of 1, 2-polybutadiene as described above, a tube formed of a blend of 1,2-polybutadiene and a styrene-isoprene copolymer (SIS), a tube formed of a blend of 1,2-polybutadiene and a rubber, and a tube formed of a blend of 1, 2-polybutadiene and an olefin resin. Of these, as for the combination with the styrene-isoprene copolymer, there may be used either hydrogenated styrene-ethylene-propylene-styrene or a partially hydrogenated product thereof. As for the combination with the rubber, various rubbers can be used, but isoprene and natural rubber are preferred. As for the combination with the olefin resin, preferred examples of the resins include LDPE, L-LDPE and EVA.

<u>Polar Resins</u>

The polar resins used in the polar resin formed article of the invention include an ABS resin, a polystyrene resin, an acrylic resin, polyacrylamide, polyacrylic acid, a polyalkyl acrylate such as polymethyl acrylate or polyethyl acrylate, polyacrylonitrile, an acrylonitrile-styrene copolymer, polymethacrylamide, polymethacrylic acid, a polyalkyl methacrylate such as a polymethyl methacrylate resin or a polyethyl methacrylate resin, a polyurethane resin, polymethacrylonitrile, an acetal resin, polyoxymethylene, an ionomer, chlorinated polyethylene, a coumarone-indene resin, regenerated cellulose, a petroleum resin, a cellulose derivative, alkali cellulose, a cellulose ester, cellulose acetate, cellulose acetate butylate, cellulose xanthate, cellulose nitrate, a cellulose ether, carboxymethylcellulose, a cellulose ether ester, a fluororesin, FEP, polychlorotrifluoroethylene, polytetrafluoroethylene, polyvinylidene fluoride, polyvinyl fluoride, a polypolyamide such as nylon 11, nylon 12, nylon 6, nylon 6, 10, nylon 6, 12, nylon 6, 6 or nylon 4, 6, an aromatic polyamide such as polyphenyleneisophthalamide, polyphenyleneterephthalamide or metaxylylenediamine, a polyimide, polyphenylene sulfide, polyether ether ketone, a polyamideimide, polyarylate, a polyester resin such as polyethylene terephthalate, polyvinyl chloride, a polyvinylidene chloride resin, chlorinated polyethylene, chlorosulfonated polyethylene, a polycarbonate, CR-39, a polysulfone, a polyethersulfone, a polysulfonamide, polyvinyl alcohol, a polyvinyl ester, polyvinyl cinnamate, polyvinyl acetate, polyvinyl ether, polyisobutyl vinyl ether, polymethyl vinyl ether, polyphenylene oxide, polybutylene terephthalate and the like as thermoplastic resins; and an amino resin, an aniline resin, a urea resin, a polysulfonamide, a melamine resin, an allyl resin, a diallyl phthalate resin, an alkyd resin, an epoxy resin, a silicone resin, a vinyl ester resin, a phenol resin, a novolac resin, a resorcinol resin, an unsaturated polyester resin, a low-shrinkage unsaturated polyester, a furan resin and the like as thermosetting resins.

Of these, the preferred polar resins include a polycarbonate resin, a polyester resin such as polyethylene terephthalate or polybutylene terephthalate, an ABS resin, a polystyrene resin, a polyacrylic resin, a polyurethane resin, a polyamide resin, a polyvinyl acetate resin, a polyvinyl chloride resin and a polyvinylidene chloride resin.

The solubility parameter (SP value) of the polar resin is preferably from 9 to 13, and more preferably from 9.5 to 12.

Here, the solubility parameter is a value calculated using the group parameter of Small by the group contributing method described in "Polymer Handbook", 4th edition, section VII, pages 682-685, published by John Wiley & Sons, Inc. (1999). For example, the parameter of polymethyl methacrylate is 9.25 $(cal/cm^3)^{1/2}$ (taking the repeating unit molecular weight as 100 g/mol, and the density as 1.19 $g/cm^3$ (hereinafter, the units are abbreviated), that of polybutyl acrylate is 8. 97 $(cal/cm^3)^{1/2}$ (taking the repeating unit molecular weight as 128, and the density as 1.06), that of polybutyl methacrylate is 9.47 $(cal/cm^3)^{1/2}$ (taking the repeating unit molecular weight as 142, and the density as 1.06), that of polystyrene is 9. 03 $(cal/cm^3)^{1/2}$ (taking the repeating unit molecular weight as 104, and the density as 1.05), and that of polyacrylonitrile is 12.71 $(cal/cm^3)^{1/2}$ (taking the repeating unit molecular weight as 53, and the density as 1.18). As the density of each polymer, there were used values described in "Ullmann's Encyclopedia of Industrial Chemistry", vol. A21, page 169, published by VCH (1992). Further, as the solubility parameter (δc) of a copolymer, the value of a main component has been used in the case where the mass fraction is less than 5%, and it has been assumed that additivity holds in mass fraction in the case where the mass fraction is 5% or more. That is to say, the parameter can be calculated by the following equation (1) from the solubility parameter δn of homopolymers of respective monomers constituting the copolymer composed of m kinds of monomers and their mass fraction Wn.

[Equation 1]

$$\delta c = \sum_{n=1}^{n=m} \delta n Wn / \sum_{n=1}^{n=m} Wn$$

For example, the solubility parameter of a copolymer composed of 75% by weight of styrene and 25% by weight of acrylonitrile is obtained as 9.95 $(cal/cm^3)^{1/2}$, with the substitution in equation (1) using 9.03 $(cal/cm^3)^{1/2}$ as the solubility parameter of polystyrene and 12.71 $(cal/cm^3)^{1/2}$ as the solubility parameter of acrylonitrile.

Further, as for the solubility parameter δs of a vinyl-based polymer obtained by polymerizing a vinyl-based monomer in two or more stages and changing the kind of vinyl-based monomer in each stage, it has been assumed that additivity holds in a value obtained by dividing the total mass of the finally obtained vinyl-based polymer by the mass of the vinyl-based polymer obtained in each stage, namely in mass fraction. That is to say, when polymerization is conducted in q stages, the parameter can be calculated by the following equation (2) from the solubility parameter δi of the polymer obtained in each stage and its mass fraction Wi.

[Equation]

$$\delta s = \sum_{i=1}^{i=q} \delta i W i / \sum_{i=1}^{i=q} W i$$

For example, when it is assumed that polymerization is conducted in two stages, that 50 parts by weight of a copolymer composed of 75% by weight of styrene and 25% by weight of acrylonitrile is obtained in the first stage, and that 50 parts by weight of a polymer of methyl methacrylate is obtained in the second stage, this polymer obtained by polymerization in two stages is obtained as 9.60 $(cal/cm^3)^{1/2}$, with the substitution in equation (2) using 9.95 $(cal/cm^3)^{1/2}$ as the solubility parameter of the styrene (75% by weight)-acrylonitrile (25% by weight) copolymer and 9.25 $(cal/cm^3)^{1/2}$ as the solubility parameter of polymethyl methacrylate.

When the solubility parameter is within the above-mentioned range, the water contact angle comes close to that of the polybutadiene composite formed article reduced in solubility parameter by ozone treatment, electron beam treatment, corona discharge treatment, plasma discharge treatment, ultraviolet treatment, ultraviolet laser treatment and chemical treatment, thereby achieving the effect of obtaining high adhesion strength in solvent adhesion with a polar solvent.

When the solubility parameter is less than 9, the adhesion force with the polar solvent unfavorably becomes insufficient. On the other hand, exceeding 13 results in strong drug adsorption, so that it is unsuitable for connector application. The polar resins which satisfy such a solubility parameter include the above-mentioned preferred polar resins.

The polar resin formed articles used in the invention include a connector, an auxiliary instrument for an infusion set, and the like, which are formed of the above-mentioned various polar resins.

The method for adhering the polybutadiene formed article of the invention comprises the steps of (1) first subjecting the polybutadiene formed article to ozone treatment, thereby introducing polar groups described later on a surface of the polybutadiene formed article, and (2) adhering the ozone-treated polybutadiene formed article to the polar resin formed article.

<u>Step (1) (Water Contact Angle-Reducing Step)</u>

Step (1) may be any process, as long as it is a means for reducing the water contact angle on the surface of the polybutadiene formed article. Examples thereof include ozone treatment, corona discharge treatment, plasma discharge treatment, excimer laser treatment, electron beam treatment, ultraviolet treatment or chemical treatment.

Ozone Treatment:

The ozone treatment is performed by exposing the polybutadiene formed article to ozone. The exposing method can be performed by an appropriate method such as a method of holding the polybutadiene formed article for a specific period of time in an atmosphere in which ozone is present or a method of exposing it for a specific period of time in an ozone flow.

Here, ozone can be generated by supplying an oxygen-containing gas such as air, oxygen gas or oxygen-added air to an ozone generating apparatus (such as an ultraviolet irradiation apparatus). The resulting ozone-containing gas is introduced into a container, a tank or the like in which the polybutadiene formed article is held, thereby performing the ozone treatment. Various conditions such as the ozone concentration in the ozone-containing gas, the exposing time and the exposing temperature can be appropriately defined according to the kind of polar resin formed article and the purpose of surface modification.

The conditions of the ozone treatment vary depending on the shape of the polybutadiene formed article, and the like. Using a flow of oxygen or air, ozone having a concentration of 1 to 200 mg/l is generated at a flow rate of 20 to 2,000 ml/min, and the treatment can be performed at a temperature of 0 to 80°C for a time of 1 minute to 24 hours. For example, the treatment at an ozone concentration of 10 to 80 mg/l at room temperature for about 20 to about 30 minutes is appropriate. Further, in the case of a film shape, the treatment at an ozone concentration of 1 to 20 mg/l at room temperature for about 30 minutes to about 6 hours is appropriate. The generated ozone concentration at the time when the air is used is about 50% of that at the time when oxygen is used.

The ozone treatment introduces percarbonic acid groups (-C-O-OH) to the surface of the polybutadiene formed

article by reaction mainly comprising oxidation, and it is presumed that parts thereof change to functional groups such as hydroxyl groups (-OH) or carbonyl groups (C=O).

Electron Beam Treatment:

The electron beam treatment is performed by using an electron beam irradiation apparatus equipped with an electron beam accelerator in place of the above-mentioned ozone generating apparatus, and irradiating the surface of the polybutadiene formed article to be treated, with electron beams generated by the electron beam accelerator. As the above-mentioned electron beam irradiation apparatus, there can be used, for example, an apparatus which can emit uniform electron beams in a curtain form from linear filaments (for example, an electrocurtain type apparatus) or the like. The dose of electron beam irradiation at this time is usually 0.5 Mrad or more, preferably 1.5 Mrad or more, and more preferably 3 Mrad or more. The dose of electron beam irradiation is set to the line speed of a treatment film on an inlet side of the electron beam irradiation apparatus, and the upper limit thereof is not particularly limited. However, it is usually about 20 Mrad.

Corona Discharge Treatment:

The corona discharge treatment is performed by using, for example, a known corona discharge treating apparatus, and allowing the polybutadiene formed article to be treated to pass through a corona atmosphere generated in an inert gas. The corona discharge density at this time is usually 10 ($w \cdot min/m^2$) or more, and preferably from 30 to 300 ($w \cdot min/m^2$). The above-mentioned inert gases include argon, helium, krypton, neon, xenon and nitrogen as a simple substance, and a mixed gas of two or more thereof. In particular, nitrogen is industrially preferred. The above-mentioned inert gas may contain oxygen at an oxygen concentration of 1% by volume or less, preferably 0.1% by volume or less and more preferably 0.01% by volume or less.

Plasma Treatment:

As the plasma treatment, there are low-pressure plasma treatment and atmospheric plasma treatment. The low-pressure plasma treatment can be performed by electronically exciting the above-mentioned inert gas with a plasma jet in a low-pressure state of 0.1 to 5 Torr at an output of 200 to 1, 000 W, then removing charged particles to make it electrically neutral, thus obtaining an exited inert gas, and bringing the exited inert gas into contact with the polybutadiene formed article to be treated. The treatment time at this time is from 10 to 60 seconds, and preferably from 20 to 40 seconds.

Further, the atmospheric plasma treatment can be performed by applying an alternating current of 2 to 3,000 V at 3 to 5 kHz between electrodes in the above-mentioned inert gas to generate an excited inert gas similar to that in the low pressure plasma treatment, and then bringing the exited inert gas into contact with the polybutadiene formed article to be treated. The treatment time at this time is from 10 to 60 seconds, and preferably from 20 to 40 seconds.

Ultraviolet Laser Treatment:

As an ultraviolet laser, there is a laser having an oscillation wavelength of 180 to 360 nm, preferably 190 to 250 nm, and preferred is an excimer laser. Gases used in the excimer laser include KrF, KrCl, ArF, ArCl, $F_2$ and the like, each of which has a specific oscillation wavelength. For example, an ArF laser has an oscillation wavelength of 192 nm, and the photon energy thereof is 148 Kcal. Accordingly, it can sever a C-H bond having a bond energy of 100 to 110 Kcal, and hydrogen excited by excimer laser irradiation can be easily drawn out. In place thereof, for example, a carbonyl group, a carboxyl group, a hydroxyl group or the like can be introduced by oxygen in the coexisting air, or the like.

The dose of the excimer laser is from 15 to 25 $mJ/cm^2$, and preferably from 18 to 22 $mJ/cm^2$. That is to say, when the energy density is less than 15 $mJ/cm^2$, it is difficult to obtain the desired hydrophilicity. On the other hand, even when it exceeds 25 $mJ/cm^2$, not only the hydrophilicity is not improved any more, but also the surface of the polybutadiene formed article as a substrate is roughened.

Chemical Treatment:

The chemical treatment is treatment of etching or deteriorating the polybutadiene formed article or treatment of introducing a functional group onto a surface thereof. Specific examples thereof include peroxide treatment of the foamed article with hydrogen peroxide or the like, treatment with an inorganic acid such as nitric acid, hydrochloric acid, sulfuric acid, chromic acid, a potassium permanganate solution or the like, treatment with a toluene solution of aluminum chloride or iron chloride, and the like.
As for conditions of the chemical treatment, the polybutadiene formed article is immersed in an acidic, neutral or basic

solvent (including water) for 5 minutes to 48 hours with 2 to 50% by weight of any one of nitric acid, hydrochloric acid, sulfuric acid, chromic acid and potassium permanganate to be oxidized. A technique of heating at 30 to 50°C can also be used as needed.

In the above-mentioned step (1), the above-mentioned ozone treatment, electron beam treatment, corona discharge treatment, plasma discharge treatment, ultraviolet laser treatment and chemical treatment can be used alone or in combination.

For example, in step (1), it is preferred in terms of reducing the water contact angle of the polybutadiene formed article that the ultraviolet laser treatment is performed under the presence of ozone.

The water contact angle of the surface of the polybutadiene formed article can be decreased by the above-mentioned step (1).

The reason for this is presumed that the water contact angle is decreased by introducing, for example, a functional group such as a carbonyl group, a carboxyl group, a hydroxyl group or an aldehyde group onto the surface of the polybutadiene formed article or roughening the surface, as described above.

Here, the water contact angle can be determined by measuring the contact angle at the time when one drop of water is gently placed on the treated tabular polybutadiene formed article, with a commercially available automatic contact angle meter, for example, an automatic contact angle meter manufactured by Kyowa Interface Science Co., Ltd.

The water contact angle of the polybutadiene formed article treated in step (1) is usually 80 degrees or less, preferably 75 degrees or less, and more preferably from 10 degrees to 70 degrees. Exceeding 80 degrees results in poor adhesion to the polar resin formed article, and causes the problem that no polar solvent can be used.

The water contact angle of the above-mentioned polar resin is usually from 80 degrees to 20 degrees, preferably from 78 degrees to 30 degrees, and more preferably from 75 degrees to 40 degrees.

Accordingly, the difference (ΔCA) between the water contact angle ($CA_{BR}$) of the water contact angle-reduced polybutadiene formed article obtained in step (1) and the water contact angle ($GA_{PR}$) of the polar resin formed article is usually from +60 degrees to -15 degrees, preferably from +60 degrees to -10 degrees, more preferably from +60 degrees to -5 degrees, and particularly preferably from +50 degrees to 0 degrees.

Although the solubility parameter of polybutadiene itself is usually from 8.3 to 8.5, and preferably 8.4, the solubility parameter of the polybutadiene formed article which has been treated in step (1) increases to usually 9.0 to 12.0, and preferably 9.3 to 11.0, coming close to the solubility parameter of the above-mentioned polar resin. Accordingly, it can also be said that adhesion properties are improved by allowing the solubility parameter of polybutadiene to approximate that of the polar resin.

The above-mentioned step (1) is applied to the polybutadiene formed article when the polybutadiene formed article and the polar resin formed article are adhered to each other. In addition to this, however, it can also be applied to the polar resin formed article.

In this case, conditions of each treatment described above to the polar resin formed article are similar to those to the polybutadiene formed article.

Step (2) (Adhesion Step)

In the invention, subsequently, the polybutadiene formed article reduced in water contact angle by the treatment of step (1) is adhered to the polar resin formed article.

Adhesion methods include solvent adhesion, ultrasonic adhesion, high-frequency adhesion, adhesion using an adhesive (including an UV cure acrylic instant adhesive and a cyanoacrylate instant adhesive) and the like, which do not impair transparency at the time of adhesion, and preferred is solvent adhesion.

Here, in the solvent adhesion, the polybutadiene formed article and the polar resin formed article are adhered to each other using an organic solvent soluble in the polybutadiene formed article and/or the polar resin formed article.

In the solvent adhesion, the organic solvent common to the polybutadiene formed article and the polar resin formed article may be used, or the solvents soluble in the respective articles may be individually used.

The above-mentioned organic solvents for adhesion include tetrahydrofuran, cyclohexane, toluene, cyclohexanone, methyl ethyl ketone, acetone, ethyl acetate and the like.

This adhesion can be performed by means of immersing adhering portions of the polybutadiene formed article and polar resin formed article in the solvent for adhesion, spraying the solvent for adhesion thereto, or applying the solvent for adhesion thereto with a brush, a waste piece from cutting cloth or the like.

In each adhesion described above, it is desirable to use the organic solvent common in the above-mentioned adhesion or the individual organic solvents in combination, and to previously treat the polybutadiene formed article and the polar resin formed article, respectively.

According to the invention, the polybutadiene formed article such as a tube treated by the ozone treatment or the like in step (1) is adhered to the polar resin formed article such as a connector, thereby obtaining the polybutadiene composite formed article in which the adhering portions are strongly adhered to each other.

An infusion set using the polybutadiene composite formed article (medical members: a tube and a connector having a tube connecting portion) of the invention will be illustrated in greater detail below with reference to Fig. 1.
The infusion set 10 comprises a connecting member (connector) 15 for connection with a tube 14 for discharging an infusion solution in an infusion bag 12, a first tube T1 for connecting the connecting member 15 to a drip chamber 11, a second tube T2 for connecting the drip chamber 11 to a puncture needle 13, a roller clamp 18 for adjusting the infusion rate and a cap 16 for covering the puncture needle 13. The reference numeral 19 designates a connecting member for connecting the second tube T2 to the puncture needle 13.
As the puncture needle 13, there is used a hollow metal needle made of stainless steel or a hollow needle made of a synthetic resin, which has a cutting edge for puncture at its tip. Further, as the roller clamp 18, there has been used a roller clamp to which a roller 17 is movably attached. The movement of the roller 17 to the side of the puncture needle 13 narrows a flow path of the second tube T2, thereby being able to adjust the infusion rate. For an emergency that foreign matter is contained in a transfusion, a filter (not shown) is contained in the drip chamber 11. As the puncture needle 13, there is used one which has hitherto been used.
Further, in the invention, all of the connecting member 15, the drip chamber 11 and the connector 19 correspond to "the connector having a tube connecting portion", and a polar resin such as a polycarbonate, a polyester, a transparent ABS or vinyl chloride is used.
Further, as the tubes T1 and T2, soft tubes having transparency are suitable. Specifically, there are used a soft vinyl chloride resin and syndiotactic 1,2-polybutadiene having a low crystallinity of about 5 to about 25% which have hitherto been used, and further, one obtained by ozone treatment of the syndiotactic 1,2-polybutadiene of the invention having a crystallinity of 5% or more, preferably a low crystallinity of about 5 to about 25%.
Respective ends of the tubes T1 and T2 are firmly adhered and fixed to the tube connecting portions of the connecting member 15, the drip chamber 11 and the connector 19 (all corresponding to the connector in the invention) by solvent adhesion, ultrasonic adhesion or high-frequency adhesion.
In the invention, tubes formed of the ozone-treated syndiotactic 1,2-polybutadiene are adhered to the connectors formed of a polycarbonate, so that both can be firmly adhered and fixed to each other, which causes no leak.
Solvents used herein for solvent adhesion include tetrahydrofuran, cyclohexane, cyclohexanone, methyl ethyl ketone, acetone, ethyl acetate, toluene and the like, as described above.
In the invention, the medical member comprising the tube(s) and the connector(s) having the tube connecting portion (s) can also be applied as a constituent element for the above-mentioned infusion set or a constituent element for the medical instrument such as a catheter for pharmaceutical administration.

## Examples

The present invention will be illustrated in greater detail with reference to the following examples, but the invention should not be construed as being limited thereto. In the examples, parts and percentages are by weight, unless otherwise specified. Further, various measurements in the examples were made according to the following:

### Transparency of Sheet

Molding temperature: 150°C
Mold temperature: 20°C

Judged by the haze of a 2-mm thick injection molded sheet.
The haze was measured with a haze meter manufactured by BYK Gardner, Inc.
Judgment

Less than 3: ○ Good (having commercial value)
3 or more to less than 5: Δ
5 or more: × Poor (having no commercial value)

### 300% Tensile Stress (Evaluation of Flexibility)

A 2-mm thick press molded sheet prepared at a molding temperature of 150°C was stamped out with a JIS No. 3 dumbbell, and the stress thereof was measured by using a universal tensile tester AG10kNE manufactured by Shimadzu Corporation to judge flexibility.
Judgment

Less than 5 MPa: ○ Good (absorbing vibration by flexibility, and therefore having commercial value)
5 MPa or more to less than 10 MPa: Δ
10 MPa or more: × Poor (poor in flexibility to transmit vibration, and therefore having no commercial value)

Preparation of Test Pieces for Ozone Treatment and Adhesion Test

Using an injection molding machine N-100 manufactured by the Japan Steel Works, Ltd., molded articles of 20 mm wide by 100 mm long by 2 mm thick were each molded at a molding temperature of 130°C/a mold temperature of 20°C for 1,2-polybutadiene [RB810 manufactured by JSR Corporation (1,2-vinyl bond content: 90%, density: $0.901 \times 10^3$ kg/m$^3$)], at a molding temperature of 270°C/a mold temperature of 30°C for a polyester resin (Easter DN010 manufactured by Eastman Chemical Company), at a molding temperature of 270°C/a mold temperature of 30°C for a polycarbonate resin (Iupilon S-3000R manufactured by Mitsubishi Engineering-Plastics Corporation), and at a molding temperature of 240°C/a mold temperature of 30°C for an ABS resin (Techno ABS810 manufactured by Techno Polymer Co., Ltd.), and used as test pieces for an adhesion test.

Ozone Treatment

Using a low-pressure mercury lamp type OC2507 (25 W, 7 lamps) manufactured by Iwasaki Electric Co. , Ltd. , a test piece (20 mm wide by 100 mm long by 2 mm thick) of RB810 manufactured by JSR Corporation, which had previously been degreased, was ozone treated. In the ozone treatment, the distance from the mercury lamp to a surface of the test piece was set to 20cm, and the irradiation time was set to 3 minutes. At this time, the intensity of an ultraviolet ray having a wavelength of 254 nm is 18mW/cm$^2$, and the integrated amount of light is 7J/cm$^2$.

Measurement of Contact Angle

Using a goniometer type contact angle measuring instrument type G1 manufactured by Elma, the contact angle of 1 μl of distilled water dropped on the test piece was measured.

Adhesion Test

On the test piece immediately after the ozone treatment and the untreated test piece (RB810 manufactured by JSR Corporation, 20 mm wide by 100 mm long by 2 mm thick), 1 ml of a specified solvent was dropped from a syringe, and each test piece was superimposed on each of various kinds of connector pieces (20 mm wide by 100 mm long by 2 mm thick) previously degreased to perform solvent adhesion. The two molded pieces were superimposed at solvent adhering portions so that an end portion of each test piece forms a rectangle of 25 mm × 12.5 mm. The test piece subjected to the solvent adhesion was heat treated at 50°C for 24 hours, and then, the temperature thereof was turned back to room temperature, followed by still standing for 2 hours. Thereafter, the shearing strength thereof was measured by using a universal tensile tester AG10kNE manufactured by Shimadzu Corporation.
Judgment

8 kgf/cm$^2$ or more: ○ Good (having commercial value)
7 or more to less than 8 kgf/cm$^2$: Δ
Less than 7 kgf/cm$^2$: × Poor (having no commercial value)

Drug Adsorption Test

Nitroglycerine (concentration: 60 μg/ml) was selected as a drug, and the drug residual ratio after an elapse of 1 hour at the time when it was allowed to pass through an infusion tube at a flow rate of 70 ml/hr was measured.
Judgment

Residual ratio 95% or more: ○ Good (having commercial value)
Less than 95% to 80% or more: Δ
Less than 80%: × Poor (having no commercial value)
Tubes subjected to the test

RB810 (manufactured by JSR Corporation) and PVC (101EP manufactured by Zeon Corporation/dioctyl phthalate/epoxy auxiliary plasticizer/organic tin stabilizer/zinc stearate =100/46/8/2/1) were each extruded with a labo extruder (Ikegai FS40 (40-mm single screw extruder (L/D=28)) of JSR research institute to prepare tubes (external diameter/internal

diameter/thickness = 3.7 mm/3 mm/0.7 mm).

Example 1 and Comparative Example 1

Example 1 and Comparative Example 1 are shown in Table 1. In Example 1, the ozone treatment was applied to the test piece of RB. It is shown that the absorption of the carbonyl group is present at 1, 720 $cm^{-1}$. It is also shown that the contact angle is improved as low as 32 degrees because of the functional group imparted, which makes it easy to adapt the test piece to cyclohexanone as the polar solvent, resulting in the possibility of adhesion to the polycarbonate as the polar resin and sufficient expression of adhesion force.
In Comparative Example 1, no ozone treatment was applied to the test piece of RB. It is therefore shown that the compatibility with the polar solvent and the polar resin is unsatisfactory, resulting in insufficient adhesion force.

Example 2 and Comparative Example 2

Example 2 and Comparative Example 2 are shown in Table 1. In Example 2, the ozone treatment was applied to the test piece of RB. It is shown that the absorption of the carbonyl group is present at 1, 720 $cm^{-1}$. It is also shown that the contact angle is improved as low as 32 degrees because of the functional group imparted, which makes it easy to adapt the test piece to cyclohexanone as the polar solvent, resulting in the possibility of adhesion to the transparent ABS as the polar resin and sufficient expression of adhesion force.
In Comparative Example 2, no ozone treatment was applied to the test piece of RB. It is therefore shown that the compatibility with the polar solvent and the polar resin is unsatisfactory, resulting in insufficient adhesion force.

Example 3 and Comparative Example 3

Example 3 and Comparative Example 3 are shown in Table 1. In Example 3, the ozone treatment was applied to the test piece of RB. It is shown that the absorption of the carbonyl group is present at 1, 720 $cm^{-1}$. It is also shown that the contact angle is improved as low as 32 degrees because of the functional group imparted, which makes it easy to adapt the test piece to cyclohexanone as the polar solvent, resulting in the possibility of adhesion to the polyester as the polar resin and sufficient expression of adhesion force.
In Comparative Example 3, no ozone treatment was applied to the test piece of RB. It is therefore shown that the compatibility with the polar solvent and the polar resin is unsatisfactory, resulting in insufficient adhesion force.

Example 1 and Comparative Example 4

Example 1 and Comparative Example 4 are shown in Table 1. In Example 1, the ozone treatment was applied to the test piece of RB. It is shown that the absorption of the carbonyl group is present at 1, 720 $cm^{-1}$. It is also shown that the contact angle is improved as low as 32 degrees because of the functional group imparted, which makes it easy to adapt the test piece to cyclohexanone as the polar solvent, resulting in the possibility of adhesion to the polycarbonate as the polar resin and sufficient expression of adhesion force. It is further shown that the test piece has no drug adsorptivity which is important for the infusion tube application, so that it has no drug loss.
In Comparative Example 4, PVC was used. It has the good compatibility with the polar solvent and the polar resin even when no ozone treatment is applied, and is excellent in adhesion strength. However, it has high drug adsorptivity which is important as an infusion tube, so that the residual drug amount is unfavorably decreased (there is drug loss). Further, a plasticizer contained in PVC has the possibility of causing harm of various kinds to the human body. This is therefore unfavorable.

Example 1 and Comparative Example 5

Example 1 and Comparative Example 5 are shown in Table 1. In Example 1, the ozone treatment was applied to the test piece of RB. It is shown that the absorption of the carbonyl group is present at 1, 720 $cm^{-1}$. It is also shown that the contact angle is improved as low as 32 degrees because of the functional group imparted, which makes it easy to adapt the test piece to cyclohexanone as the polar solvent, resulting in the possibility of adhesion to the polycarbonate as the polar resin and sufficient expression of adhesion force. It is further shown that the test piece has transparency (a proper flow of a drug solution can be confirmed) and flexibility (300% tensile stress: flexibility preferably makes it difficult to transmit shock generated by contact with a tube to a needle connected to the human body) which are important for the infusion tube application, so that it has excellent characteristics.
In Comparative Example 5, PE was used. It has no polarity, so that it is poor in the compatibility with the polar solvent and the polar resin, resulting in poor adhesion strength. Further, it is poor in transparency and flexibility which are

important as an infusion tube. This is therefore unfavorable.

[Table 1]

| | Example 1 | Comparative Example 1 | Example 2 | Comparative Example 2 |
|---|---|---|---|---|
| 1. Tube Formed Article<br>2. Connector<br>3. Adhesion Solvent | RB<br>Treated<br>Polycarbonate<br>Cyclohexanone | RB<br>Untreated<br>Polycarbonate<br>Cyclohexanone | RB<br>Treated<br>Transparent ABS<br>Cyclohexanone | RB<br>Untreated<br>Transparent ABS<br>Cyclohexanone |
| [Results of Evaluation] | | | | |
| Contact Angle between (RB) Formed Article and Water (degrees) | 32 | 93 | 32 | 93 |
| Adhesion Strength ($kgf/cm^2$) | 12.2 | 6.8 | 8.4 | 4.8 |
| Drug Adsorption Residual Ratio Just after Elapse (%) | 100 | | | |
| Transparency Haze (%) | 2.6 | | | |
| 300% Tensile Stress (MPa) | 3.9 | | | |

| | Example 3 | Comparative Example 3 | Comparative Example 4 | Comparative Example 5 |
|---|---|---|---|---|
| 1. Tube Formed Article<br>2. Connector<br>3. Adhesion Solvent | RB<br>Treated<br>Polyester<br>Cyclohexanone /THF Mixed Solvent | RB<br>Untreated<br>Polyester<br>Cyclohexanone /THF Mixed Solvent | PVC<br>Polycarbonate<br>Cyclohexanone | PE<br>Polycarbonate<br>Cyclohexanone |
| [Results of Evaluation] | | | | |
| Contact Angle between (RB) Formed Article and Water (degrees) | 32 | 93 | 66 | 96 |
| Adhesion Strength ($kgf/cm^2$) | 15.3 | 7.4 | 12 | 2.2 |
| Drug Adsorption Residual Ratio Just after Elapse (%) | | | 70 | |
| Transparency Haze (%) | | | | 8.7 |
| 300% Tensile Stress (MPa) | | | | 70 |

Industrial Applicability

According to the invention, there can be provided a medical member mainly comprising 1,2-polybutadiene, which is useful for medical use, is excellent in flexibility and hardness, is also excellent in steam sterilization resistance, has no leak at a joint, is recyclable, and is further environmentally friendly because it contains no vinyl chloride-based resin, and a medical instrument using the same.

## Claims

**1.** A method for adhering a polybutadiene formed article, which comprises the steps of:

(1) reducing the water contact angle of a surface of the polybutadiene formed article, and
(2) adhering the polybutadiene formed article which is reduced in the water contact angle to a polar resin formed article.

2. The method for adhering a polybutadiene formed article according to claim 1, wherein the polybutadiene is syndiotactic 1,2-polybutadiene having a crystallinity of 5% or more.

3. The method for adhering a polybutadiene formed article according to claim 1 or 2, wherein step (1) is at least one selected from the group consisting of ozone treatment, electron beam treatment, corona discharge treatment, plasma discharge treatment, ultraviolet laser treatment and chemical treatment.

4. The method for adhering a polybutadiene formed article according to any one of claims 1 to 3, wherein the water contact angle ($CA_{BR}$) of the water contact angle-reduced polybutadiene formed article which is obtained in step (1), is 80 degrees or less.

5. The method for adhering a polybutadiene formed article according to any one of claims 1 to 4, wherein the polar resin is at least one selected from the group consisting of a polycarbonate resin, a polyester resin, an ABS resin, a polystyrene resin, a polyurethane resin, a polyamide resin, a polyalkyl acrylate resin, a polyalkyl methacrylate resin, a polyvinyl acetate resin, a polyvinyl chloride resin and a polyvinylidene chloride resin.

6. The method for adhering a polybutadiene formed article according to any one of claims 1 to 5, wherein the difference ($\Delta CA$) between the water contact angle ($CA_{BR}$) of the water contact angle-reduced polybutadiene formed article obtained in step (1) and the water contact angle ($CA_{PR}$) of the polar resin formed article is from +60 degrees to -15 degrees.

7. The method for adhering a polybutadiene formed article according to any one of claims 1 to 6, wherein the adhesion in step (2) is preferably adhesion by the use of an organic solvent.

8. The method for adhering a polybutadiene formed article according to any one of claims 1 to 7, wherein the organic solvent is at least one selected from the group consisting of cyclohexanone, tetrahydrofuran, cyclohexane, methyl ethyl ketone, acetone and ethyl acetate.

9. The method for adhering a polybutadiene formed articleaccording to any one of claims 1 to 8, wherein the water contact angle-reduced polybutadiene formed article which is obtained in step (1) and the polar resin formed article are previously treated with the organic solvent according to claim 8.

10. A polybutadiene composite formed article obtained by the method according to any one of claims 1 to 9.

11. A medical member comprising at least the polybutadiene composite formed article according to claim 10.

12. An infusion set having the medical member according to claim 11 as a constituent element.

Figure 1

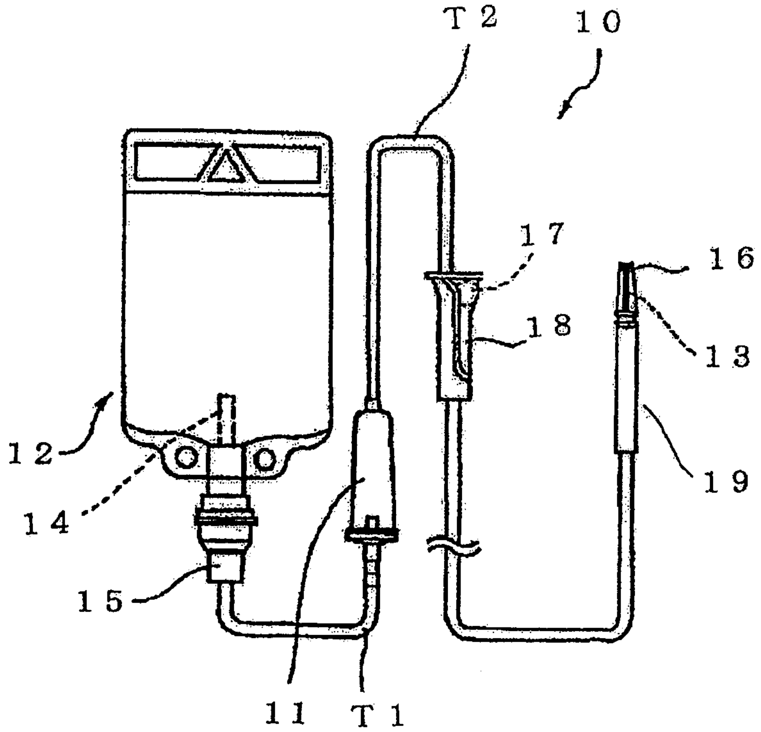


Figure 2

(a)

(b)

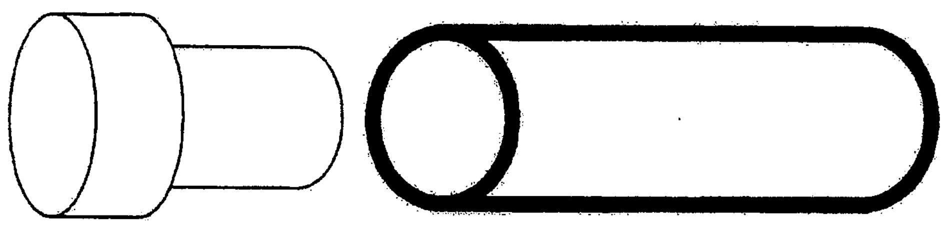

**INTERNATIONAL SEARCH REPORT**

International application No.
PCT/JP2004/019296

A. CLASSIFICATION OF SUBJECT MATTER
Int.Cl[7] C08J5/12, C08J7/00, C08J7/02, A61M5/14//C08L9:00

According to International Patent Classification (IPC) or to both national classification and IPC

B. FIELDS SEARCHED

Minimum documentation searched (classification system followed by classification symbols)
Int.Cl[7] C08J5/12, C08J7/00, C08J7/02, A61M5/14, C08L9/00

Documentation searched other than minimum documentation to the extent that such documents are included in the fields searched

| | | | |
|---|---|---|---|
| Jitsuyo Shinan Koho | 1926-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2001-162723 A (Tokai Rubber Industries, Ltd.),<br>19 June, 2001 (19.06.01),<br>Claims; pages 4 to 5, Par. No. [0023]; page 6, Par. No. [0034]<br>(Family: none) | 1-6<br>7-12 |
| Y | WO 2003/018685 A2 (BAXTER INTERNATIONAL INC.),<br>06 March, 2003 (06.03.03),<br>Claims; page 12, lines 1 to 26; pages 17 to 18; example 7; table 6<br>& AU 2002356125 A1 & EP 1423473 A2<br>& KR 2004029437 A & NO 200401223 A<br>& US 2003/0044555 A1 | 1-12 |

[X] Further documents are listed in the continuation of Box C.    [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 February, 2005 (10.02.05) | Date of mailing of the international search report<br>01 March, 2005 (01.03.05) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2004/019296

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-190274 A (Nippon Zeon Co., Ltd.), 08 July, 2003 (08.07.03), Claims; page 3, Par. Nos. [0014] to [0015], [0017] to [0018] (Family: none) | 1-12 |
| Y | JP 2000-245851 A (Terumo Corp.), 12 September, 2000 (12.09.00), Claims; page 5, Par. Nos. [0027] to [0028]; pages 5 to 6, Par. Nos. [0034] to [0035] (Family: none) | 1-12 |

Form PCT/ISA/210 (continuation of second sheet) (January 2004)